(19) Europäisches Patentamt
European Patent Office
Office européen des brevets

(11) Veröffentlichungsnummer: **0 434 992 A1**

(12) **EUROPÄISCHE PATENTANMELDUNG**

(21) Anmeldenummer: **90122552.4**

(22) Anmeldetag: **26.11.90**

(51) Int. Cl.[5]: **C07K 13/00**, C12N 15/41, C12P 21/00, G01N 33/569

(30) Priorität: **27.11.89 DE 3939200**

(43) Veröffentlichungstag der Anmeldung: **03.07.91 Patentblatt 91/27**

(84) Benannte Vertragsstaaten: **AT BE CH DE DK ES FR GB GR IT LI LU NL SE**

(71) Anmelder: **Max-Planck-Gesellschaft zur Förderung der Wissenschaften e.V.**
**Bunsenstrasse 10**
**W-3400 Göttingen(DE)**

(72) Erfinder: **Kandolf, Reinhard, Dr.**
**Riesstrasse 64**
**W-8000 München 50(DE)**

(74) Vertreter: **Huber, Bernhard, Dipl.-Chem. et al**
**Patentanwälte H. Weickmann, K. Fincke, F.A. Weickmann, B. Huber, H. Liska, J.Prechtel, Möhlstrasse 22**
**W-8000 München 80(DE)**

(54) **Enterovirale Polypeptide und gruppenspezifischer Nachweis von Enterovirusinfektionen.**

(57) Es wird ein Polypeptid beschrieben, das für die Gruppe der Enteroviren spezifisch ist und zum Nachweis von Infektionen mit diesen Viren eingesetzt werden kann.

EP 0 434 992 A1

# ENTEROVIRALE POLYPEPTIDE UND GRUPPENSPEZIFISCHER NACHWEIS VON ENTEROVIRUSINFEKTIONEN

Die Erfindung betrifft Polypeptide, DNA-Sequenzen, welche für diese Polypeptide kodieren, die Verwendung dieser Polypeptide und einen gruppenspezifischen Test zum Nachweis von Enteroviren sowie enteroviralen Antikorpern.

In der klinischen. Praxis spielt die Serodiagnose viraler Infektionen eine zentrale Rolle, die jedoch im Falle von Infektionen mit Enteroviren, welche die häufigste Ursache der viralen Myocarditis des Menschen darstellen, aufgrund der Vielzahl cardiotroper Enteroviren mit über 70 verschiedenen Serotypen ein bislang ungelöstes Problem darstellt. Die Enteroviren sind eine Gruppe von Viren, die zunächst den Respirations- oder Gastrointestinaltrakt infizieren, sich dort vermehren und sodann im Falle einer Virämie verschiedene Zielorgane infizieren, wie z.B. das Herz, das Pankreas, das zentrale Nervensystem oder das Rückenmark. Beispiele für diese Viren sind das Poliovirus, das Echovirus und das Coxsackie-Virus. Typische Erkrankungen nach Infektion mit solchen Viren sind Myocarditis, Pankreatitis und Meningitis. Die Enteroviren gehören zu der Gruppe der RNA-Viren.

Im Hinblick auf die Häufigkeit gerade von viralen Herzerkrankungen kommt der Gruppe der Enteroviren eine sehr große klinische Bedeutung zu. Der Nachweis von Enterovirus-Infektionen als Ursache viraler Herzkrankheiten oder die prophylaktische Untersuchung bei Verdacht auf eine Enterovirus-Infektion machte bisher außerordentliche Schwierigkeiten. Eine spezifische Diagnose und damit eine Bestätigung eines klinischen Verdachts auf eine virale Herz-, oder andere Erkrankung nach Methoden des bisherigen Standes der Technik ist außerordentlich schwierig. Hierzu wurden bisher nämlich Hyperimmunseren eingesetzt, die gegen gereinigte Viruspartikel einzelner Serotypen hergestellt wurden. Es war damit nur möglich, jeweils genau diejenigen Viren nachzuweisen, gegen die die Hyperimmunseren erzeugt worden waren. Da jedoch, wie bereits oben ausgeführt, über 70 verschiedene Serotypen kardiotroper Enteroviren existieren, war hierdurch ein umfassender Erregernachweis nicht möglich. Obwohl eine Reihe von antigenen Kreuzreaktionen zwischen verschiedenen Enteroviren beschrieben wurde, war bislang eine Enterovirus-gruppenspezifische Serodiagnostik aufgrund der antigenen Heterogenität zwischen und innerhalb distinkter Serotypen überaus unbefriedigend bzw. nicht möglich. Zudem bestand bislang keine Möglichkeit zum Nachweis von Antikörpern gegen enterovirale RNA-Polymerasen.

Der Erfindung lag daher die Aufgabe zugrunde, die Voraussetzungen für einen gruppenspezifischen Nachweis, der für die gesamte Familie der Enteroviren spezifisch ist, zu schaffen, anhand dessen die An- oder Abwesenheit dieser Viren sowie deren Antikörper im Blut oder Serum von Patienten bestimmbar ist.

Gelöst wird diese Aufgabe durch die erfindungsgemäßen Polypeptide, nämlich mit der Sequenz

```
  MGAQVSTQ KTGAHETRLN ASGNSIIHYT NINYYKDAAS NSANRQDFTQ
DPGKFTEPVK DIMIKSLPAL NSPTVEECGY SDRARSITLG NSTITTQECA
NVVVGYGVWP DYLKDSEATA EDQPTQPDVA TCRFYTLDSV QWQKTSPGWW
WKLPDALSNL GLFGQNMQYH YLGRTGYTVH VQCNASKFHQ GCLLVVCVPE
AEMGCATLDN TPSSAELLGG DTAKEFADKP VASGSNKLVQ RVVYNAGMGV
GVGNLTIFPH QWINLRTNNS ATIVMPYTNS VPMDNMFRHN NVTLMVIPFV
PLDYCPGSTT YVPITVTIAP MCAEYNGLRL AGHQGLPTMN TPGSCQFLTS
DDFQSPSAMP QYDVTPEMRI PGEVKNLMEI AEVDSVVPVQ NVGEKVNSME
AYQIPVRSNE GSGTQVFGFP LQPGYSSVFS RTLLGEILNY YTHWSGSIKL
```

TFMFCGSAMA TGKFLLAYSP PGAGAPTKRV DAMLGTHVIW DVGLQSSCVL
CIPWISQTHY RFVASDEYTA GGFITCWYQT NIVVPADAQS SCYIMCFVSA
CNDFSVRLLK DTPFISQQNF FQGPVEDAIT AAIGRVADTV GTGPTNSEAI
PALTAAETGH TSQVVPGDTM QTRHVKNYHS RSESTIENFL CRSACVYFTE
YKNSGAKRYA EWVLTPRQAA QLRRKLEFFT YVRFDLELTF VITSTQQPST
TQNQDAQILT HQIMYVPPGG PVPDKVDSYV WQTSTNPSVF WTEGNAPPRM
SIPFLSIGNA YSNFYDGWSE FSRNGVYGIN TLNNMGTLYA RHVNAGSTGP
IKSTIRIYFK PKHVKAWIPR PPRLCQYEKA KNVNFQPSGV TTTRQSITTM
TNTGAFGQQS GAVYVGNYRV VNRHLATSAD WQNCVWESYN RDLLVSTTTA
HGCDIIARCQ CTTGVYFCAS KNKHYPISFE GPGLVEVQES EYYPRRYQSH
VLLAAGFSEP GDCGGILRCE HGVIGIVTMG GEGVVGFADI RDLLWLEDDA
MEQGVKDYVE QLGNAFGSGF TNQICEQVNL LKESLVGQDS ILEKSLKALV
KIISALVIVV RNHDDLITVT ATLALIGCTS SPWRWLKQKV SQYYGIPMAE
RQNNSWLKKF TEMTNACKGM EWIAVKIQKF IEWLKVKILP EVREKHEFLN
RLKQLPLLES QIATIEQSAP SQSDQEQLFS NVQYFAHYCR KYAPLYAAEA
KRVFSLEKKM SNYIQFKSKC RIEPVCLLLH GSPGAGKSVA TNLIGRSLAE
KLNSSVYSLP PDPDHFDGYK QQAVVIMDDL CQNPDGKDVS LFCQMVSSVD
FVPPMAALEE KGILFTSPFV LASTNAGSIN APTVSDSRAL ARRFHFDMNI
EVISMYSQNG KINMPMSVKT CDDECCPVNF KKCCPLVCGK AIQFIDRRTQ
VRYSLDMLVT EMFREYNHRH SVGTTLEALF QGPPVYREIK ISVAPETPPP
PAIADLLKSV DSEAVREYCK EKGWLVPEIN STLQIEKHVS RAFICLQALT
TFVSVAGIIY IIYKLFAGFQ GAYTGVPNQK PRVPTLRQAK VQGPAFEFAV
AMMKRNSSTV KTEYGEFTML GIYDRWAVLP RHAKPGPTIL MNDQEVGVLD
AKELVDKDGT NLELTLLKLN RNEKFRDIRG FLAKEEVEVN EAVLAINTSK
FPNMYIPVGQ VTEYGFLNLG GTPTKRMLMY NFPTRAGQCG GVLMSTGKVL
GIHVGGNGHQ GFSAALLKHY FNDEQGEIEF IESSKDAGFP VINTPSKTKL
EPSVFHQVFE GNKEPAVLRS GDPRLKANFE EAIFSKYIGN VNTHVDEYML
EAVDHYAGQL ATLDISTEPM KLEDAVYGTE GLEALDLTTS AGYPYVALGI
KKRDILSKKT KDLTKLKECM DKYGLNLPMV TYVKDELRSI EKVAKGKSRL
IEASSLNDSV AMRQTFGNLY KTFHLNPGVV TGSAVGCDPD LFWSKIPVML
DGHLIAFDYS GYDASLSPVW FACLKMLLEK LGYTHKETNY IDYLCNSHHL
YRDKHYFVRG GMPSGCSGTS IFNSMINNII IRTLMLKVYK GIDLDQFRMI
AYGDDVIASY PWPIDASLLA EAGKGYGLIM TPADKGECFN EVTWTNATFL
KRYFRADEQY PFLVHPVMPM KDIHESIRWT KDPKNTQDHV RSLCLLAWHN
GEHEYEEFIR KIRSVPVGRC LTLPAFSTLR RKWLDSF

bzw. den bevorzugten Polypeptiden, deren Teilsequenzen von Aminosäure 3 bis 448 (VP4/2/3), Aminosäure 516 bis 952 (VP1) oder Aminosäure 1769 bis 2129 (Polymerase) in den Ansprüchen 2 bis 4 angegeben sind. Die erfindungsgemäß bevorzugten Polypeptide stellen distinkte, bakteriell synthetisierte virale Strukturproteine, (VP4/2/3 bzw. VP1) sowie die RNA-abhängige RNA-Polymerase von Coxsackie-Virus B3 dar.

Überraschenderweise wurde nämlich festgestellt, daß vor allem die viralen Strukturproteine VP4/2/3 und VP1, aber auch die Polymerase zur Bildung von Antikörpern führen, welche für die gesamte Gruppe der Enteroviren spezifisch sind. Es ist nämlich erfindungsgemäß erstmals gelungen, Polypeptide mit antigenen Eigenschaften ausfindig zu machen, die offensichtlich allen der über 70 Serotypen von Enteroviren gemeinsam sind.

Eine weitere bevorzugte Ausführungsform der Erfindung sind Polypeptide, die wenigstens eine der auf den erfindungsgemäßen Polypeptiden vorhandene antigene Determinante aufweisen.

Ein weiterer Gegenstand der Erfindung ist eine rekombinante DNA, welche einligiert in einen geeigneten Expressionsvektor, eine DNA-Sequenz enthält, die für ein erfindungsgemäßes Polypeptid nach einem der Ansprüche 1 bis 5 kodiert. Als Expressionsvektor sind hierbei alle für die Expression in Wirtszellen geeigneten Moleküle verwendbar, wie Plasmide, Cosmide, Phagengenome in ihrer doppelsträngigen (RF)-Form und andere. Die Auswahl des richtigen Vektormoleküls ist hierbei von der Auswahl zur Expression zu verwendender Wirtszellen abhängig und dem Fachmann geläufig.

In einer bevorzugten Ausführungsform enthält die rekombinante DNA die folgende Sequenz:

1 UUAAAACAGC CUGUGGGUUG AUCCCACCCA CAGGCCCAUU GGGCGCUAGC
51 ACUCUGGUAU CACGGUACCU UUGUGCGCCU GUUUUAUACC CCCUCCCCCA
101 ACUGUAACUU AGAAGUAACA CACACCGAUC AACAGUCAGC GUGGCACACC
151 AGCCACGUUU UGAUCAAGCA CUUCUGUUAC CCCGGACUGA GUAUCAAUAG
201 ACUGCUCACG CGGUUGAAGG AGAAAGCGUU CGUUAUCCGG CCAACUACUU
251 CGAAAAACCU AGUAACACCG UGGAAGUUGC AGAGUGUUUC GCUCAGCACU
301 ACCCCAGUGU AGAUCAGGUC GAUGAGUCAC CGCAUUCCCC ACGGGCGACC
351 GUGGCGGUGG CUGCGUUGGC GGCCUGCCCA UGGGGAAACC CAUGGGACGC
401 UCUAAUACAG ACAUGGUGCG AAGAGUCUAU UGAGCUAGUU GGUAGUCCUC
451 CGGCCCCUGA AUGCGGCUAA UCCUAACUGC GGAGCACACA CCCUCAAGCC
501 AGAGGGCAGU GUGUCGUAAC GGGCAACUCU GCAGCGGAAC CGACUACUUU
551 GGGUGUCCGU GUUUCAUUUU AUUCCUAUAC UGGCUGCUUA UGGUGACAAU
601 UGAGAGAUCG UUACCAUAUA GCUAUUGGAU UGGCCAUCCG GUGACUAAUA
651 GAGCUAUUAU AUAUCCCUUU GUUGGGUUUA UACCACUUAG CUUGAAAGAG
701 GUUAAAACAU UACAAUUCAU UGUUAAGUUG AAUACAGCAA AAUGGGAGCU
751 CAAGUAUCAA CGCAAAAGAC UGGGGCACAU GAGACCAGGC UGAAUGCUAG
801 CGGCAAUUCC AUCAUUCACU ACACAAAUAU UAAUUAUUAC AAGGAUGCCG
851 CAUCCAACUC AGCCAAUCGG CAGGAUUUCA CUCAAGACCC GGGCAAGUUC
901 ACAGAACCAG UGAAAGAUAU CAUGAUUAAA UCACUACCAG CUCUCAACUC
951 CCCCACAGUA GAGGAGUGCG GAUACAGUGA CAGGGCGAGA UCAAUCACAU
1001 UAGGUAACUC CACCAUAACG ACUCAGGAAU GCGCCAACGU GGUGGUGGGC
1051 UAUGGAGUAU GGCCAGAUUA UCUAAAGGAU AGUGAGGCAA CAGCAGAGGA
1101 CCAACCGACC CAACCAGACG UUGCCACAUG UAGGUUCUAU ACCCUUGACU
1151 CUGUGCAAUG GCAGAAAACC UCACCAGGAU GGUGGUGGAA GCUGCCCGAU
1201 GCUUUGUCGA ACUUAGGACU GUUUGGGCAG AACAUGCAGU ACCACUACUU
1251 AGGCCGAACU GGGUAUACCG UACAUGUGCA GUGCAAUGCA UCUAAGUUCC
1301 ACCAAGGAUG CUUGCUAGUA GUGUGUGUAC CGGAAGCUGA GAUGGGUUGC
1351 GCAACGCUAG ACAACACCCC AUCCAGUGCA GAAUUGCUGG GGGGCGAUAC
1401 GGCAAAGGAG UUUGCGGACA AACCGGUCGC AUCCGGGUCC AACAAGUUGG
1451 UACAGAGGGU GGUGUAUAAU GCAGGCAUGG GGGUGGGUGU UGGAAACCUC
1501 ACCAUUUUCC CCCACCAAUG GAUCAACCUA CGCACCAAUA AUAGUGCUAC
1551 AAUUGUGAUG CCAUACACCA ACAGUGUACC UAUGGAUAAC AUGUUUAGGC
1601 AUAACAACGU CACCCUAAUG GUUAUCCCAU UUGUACCGCU AGAUUACUGC

1651 CCUGGGUCCA CCACGUACGU CCCAAUUACG GUCACGAUAG CCCCAAUGUG
1701 UGCCGAGUAC AAUGGGUUAC GUUUAGCAGG GCACCAGGGC UUACCAACCA
1751 UGAAUACUCC GGGGAGCUGU CAAUUUCUGA CAUCAGACGA CUUCCAAUCA
1801 CCAUCCGCCA UGCCGCAAUA UGACGUCACA CCAGAGAUGA GGAUACCUGG
1851 UGAGGUGAAA AACUUGAUGG AAAUAGCUGA GGUUGACUCA GUUGUCCCAG
1901 UCCAAAAUGU UGGAGAGAAG GUCAACUCUA UGGAAGCAUA CCAGAUACCU
1951 GUGAGAUCCA ACGAAGGAUC UGGAACGCAA GUAUUCGGCU UUCCACUGCA
2001 ACCAGGGUAC UCGAGUGUUU UUAGUCGGAC GCUCCUAGGA GAGAUCUUGA
2051 ACUAUUAUAC ACAUUGGUCA GGCAGCAUAA AGCUUACGUU UAUGUUCUGU
2101 GGUUCGGCCA UGGCUACUGG AAAAUUCCUU UUGGCAUACU CACCACCAGG
2151 UGCUGGAGCU CCUACAAAAA GGGUUGAUGC UAUGCUUGGU ACUCAUGUAA
2201 UUUGGGACGU GGGGCUACAA UCAAGUUGCG UGCUGUGUAU ACCCUGGAUA
2251 AGCCAAACAC ACUACCGGUU UGUUGCUUCA GAUGAGUAUA CCGCAGGGGG
2301 UUUUAUUACG UGCUGGUAUC AAACAAACAU AGUGGUCCCA GCGGAUGCCC
2351 AAAGCUCCUG UUACAUCAUG UGUUUCGUGU CAGCAUGCAA UGACUUCUCU
2401 GUCAGGCUAU UGAAGGACAC UCCUUUCAUU UCGCAGCAAA ACUUUUUCCA
2451 GGGCCCAGUG GAAGACGCGA UAACAGCCGC UAUAGGGAGA GUUGCGGAUA
2501 CCGUGGGUAC AGGGCCAACC AACUCAGAAG CUAUACCAGC ACUCACUGCU
2551 GCUGAGACGG GUCACACGUC ACAAGUAGUG CCGGGUGACA CUAUGCAGAC
2601 ACGCCACGUU AAGAACUACC AUUCAAGGUC CGAGUCAACC AUAGAGAACU
2651 UCCUAUGUAG GUCAGCAUGC GUGUACUUUA CGGAGUAUAA AAACUCAGGU
2701 GCCAAGCGGU AUGCUGAAUG GGUAUUAACA CCACGACAAG CAGCACAACU
2751 UAGGAGAAAG CUAGAAUUCU UUACCUACGU CCGGUUCGAC CUGGAGCUGA
2801 CGUUUGUCAU AACAAGUACU CAACAGCCCU CAACCACACA GAACCAAGAU
2851 GCACAGAUCC UAACACACCA AAUUAUGUAU GUACCACCAG GUGGACCUGU
2901 ACCAGAUAAA GUUGAUUCAU ACGUGUGGCA AACAUCUACG AAUCCCAGUG
2951 UGUUUUGGAC CGAGGGAAAC GCCCCGCCGC GCAUGUCCAU ACCGUUUUUG
3001 AGCAUUGGCA ACGCCUAUUC AAAUUUCUAU GACGGAUGGU CUGAAUUUUC
3051 CAGGAACGGA GUUUACGGCA UCAACACGCU AAACAACAUG GGCACGCUAU
3101 AUGCAAGACA UGUCAACGCU GGAAGCACGG GUCCAAUAAA AAGCACCAUU
3151 AGAAUCUACU UCAAACCGAA GCAUGUCAAA GCGUGGAUAC CUAGACCACC
3201 UAGACUCUGC CAAUACGAGA AGGCAAAGAA CGUGAACUUC CAACCCAGCG
3251 GAGUUACCAC UACUAGGCAA AGCAUCACUA CAAUGACAAA UACGGGCGCA
3301 UUUGGACAAC AAUCAGGGGC AGUGUAUGUG GGGAACUACA GGGUGGUAAA

3351 UAGACAUCUA GCUACCAGUG CUGACUGGCA AAACUGUGUG UGGGAAAGUU
3401 ACAACAGAGA CCUCUUAGUG AGCACGACCA CAGCACAUGG AUGUGAUAUU
3451 AUAGCCAGAU GUCAGUGCAC AACGGGAGUG UACUUUUGUG CGUCCAAAAA
3501 CAAGCACUAC CCAAUUUCGU UUGAAGGACC AGGUCUAGUA GAGGUCCAAG
3551 AGAGUGAAUA CUACCCCAGG AGAUACCAAU CCCAUGUGCU UUUAGCAGCU
3601 GGAUUUUCCG AACCAGGUGA CUGUGGCGGU AUCCUAAGGU GUGAGCAUGG
3651 UGUCAUUGGC AUUGUGACCA UGGGGGGUGA AGGCGUGGUC GGCUUUGCAG
3701 ACAUCCGUGA UCUCCUGUGG CUGGAAGAUG AUGCAAUGGA ACAGGGAGUG
3751 AAGGACUAUG UGGAACAGCU UGGAAAUGCA UUCGGCUCCG GCUUUACUAA
3801 CCAAAUAUGU GAGCAAGUCA ACCUCCUGAA AGAAUCACUA GUGGGUCAAG
3851 ACUCCAUCUU AGAGAAAUCU CUAAAAGCCU UAGUUAAGAU AAUAUCAGCC
3901 UUAGUAAUUG UGGUGAGGAA CCACGAUGAC CUGAUCACUG UGACUGCCAC
3951 ACUAGCCCUU AUCGGUUGUA CCUCGUCCCC GUGGCGGUGG CUCAAACAGA
4001 AGGUGUCACA AUAUUACGGA AUCCCUAUGG CUGAACGCCA AAACAAUAGC
4051 UGGCUUAAGA AAUUUACUGA AAUGACAAAU GCUUGCAAGG GUAUGGAAUG
4101 GAUAGCUGUC AAAAUUCAGA AAUUCAUUGA AUGGCUCAAA GUAAAAAUUU
4151 UGCCAGAGGU CAGAGAAAAA CACGAGUUCC UGAACAGACU UAAACAACUC
4201 CCCUUAUUAG AAAGUCAGAU CGCCACAAUC GAGCAGAGCG CGCCAUCCCA
4251 AAGUGACCAG GAACAAUUAU UUUCCAAUGU CCAAUACUUU GCCCACUAUU
4301 GCAGAAAGUA CGCUCCCCUC UACGCAGCUG AAGCAAAGAG GGUGUUCUCC
4351 CUUGAGAAGA AGAUGAGCAA UUACAUACAG UUCAAGUCCA AAUGCCGUAU
4401 UGAACCUGUA UGUUUGCUCC UGCACGGGAG CCCUGGUGCC GGCAAGUCGG
4451 UGGCAACAAA CUUAAUUGGA AGGUCGCUUG CUGAGAAACU CAACAGCUCA
4501 GUGUACUCAC UACCGCCAGA CCCAGAUCAC UUCGACGGAU ACAAACAGCA
4551 GGCCGUGGUG AUUAUGGACG AUCUAUGCCA GAAUCCUGAU GGGAAAGACG
4601 UCUCCUUGUU CUGCCAAAUG GUUUCCAGUG UAGAUUUUGU ACCACCCAUG
4651 GCUGCCCUAG AAGAGAAAGG CAUUCUGUUC ACCUCACCGU UUGUCUUGGC
4701 AUCGACCAAU GCAGGAUCUA UUAAUGCUCC AACCGUGUCA GAUAGCAGAG
4751 CCUUGGCAAG GAGAUUUCAC UUUGACAUGA ACAUCGAGGU UAUUUCCAUG
4801 UACAGUCAGA AUGGCAAGAU AAACAUGCCC AUGUCAGUCA AGACUUGUGA
4851 CGAUGAGUGU UGCCCGGUCA AUUUUAAAAA GUGCUGCCCU CUUGUGUGUG
4901 GGAAGGCUAU ACAAUUCAUU GAUAGAAGAA CACAGGUCAG AUACUCUCUA
4951 GACAUGCUAG UCACCGAGAU GUUUAGGGAG UACAAUCAUA GACAUAGCGU
5001 GGGGACCACG CUUGAGGCAC UGUUCCAGGG ACCACCAGUA UACAGAGAGA
5051 UCAAAAUUAG CGUUGCACCA GAGACACCAC CACCGCCCGC CAUUGCGGAC

5101 CUGCUCAAAU CGGUAGACAG UGAGGCUGUG AGGGAGUACU GCAAAGAAAA

5151 AGGAUGGUUG GUUCCUGAGA UCAACUCCAC CCUCCAAAUU GAGAAACAUG

5201 UCAGUCGGGC UUUCAUUUGC UUACAGGCAU UGACCACAUU UGUGUCAGUG

5251 GCUGGAAUCA UAUAUAUAAU AUAUAAGCUC UUUGCGGGUU UUCAAGGUGC

5301 UUAUACAGGA GUGCCCAACC AGAAGCCCAG AGUGCCUACC CUGAGGCAAG

5351 CAAAAGUGCA AGGCCCUGCC UUUGAGUUCG CCGUCGCAAU GAUGAAAAGG

5401 AACUCAAGCA CGGUGAAAAC UGAAUAUGGC GAGUUUACCA UGCUGGGCAU

5451 CUAUGACAGG UGGGCCGUUU UGCCACGCCA CGCCAAACCU GGGCCAACCA

5501 UCUUGAUGAA UGAUCAAGAG GUUGGUGUGC UAGAUGCCAA GGAGCUAGUA

5551 GACAAGGACG GCACCAACUU AGAACUGACA CUACUCAAAU UGAACCGGAA

5601 UGAGAAGUUC AGAGACAUCA GAGGCUUCUU AGCCAAGGAG GAAGUGGAGG

5651 UUAAUGAGGC AGUGCUAGCA AUUAACACCA GCAAGUUUCC CAACAUGUAC

5701 AUUCCAGUAG GACAGGUCAC AGAAUACGGC UUCCUAAACC UAGGUGGCAC

5751 ACCCACCAAG AGAAUGCUUA UGUACAACUU CCCCACAAGA GCAGGCCAGU

5801 GUGGUGGAGU GCUCAUGUCC ACCGGCAAGG UACUGGGUAU CCAUGUUGGU

5851 GGAAAUGGCC AUCAGGGCUU CUCAGCAGCA CUCCUCAAAC ACUACUUCAA

5901 UGAUGAGCAA GGUGAAAUAG AAUUUAUUGA GAGCUCAAAG GACGCCGGGU

5951 UUCCAGUCAU CAACACACCA AGUAAAACAA AGUUGGAGCC UAGUGUUUUC

6001 CACCAGGUCU UUGAGGGGAA CAAAGAACCA GCAGUACUCA GGAGUGGGGA

6051 UCCACGUCUC AAGGCCAAUU UUGAAGAGGC UAUAUUUUCC AAGUAUAUAG

6101 GAAAUGUCAA CACACACGUG GAUGAGUACA UGCUGGAAGC AGUGGACCAC

6151 UACGCAGGCC AACUAGCCAC CCUAGAUAUC AGCACUGAAC CAAUGAAACU

6201 GGAGGACGCA GUGUACGGUA CCGAGGGUCU UGAGGCGCUU GAUCUAACAA

6251 CGAGUGCCGG UUACCCAUAU GUUGCACUGG GUAUCAAGAA GAGGGACAUC

6301 CUCUCUAAGA AGACUAAGGA CCUAACAAAG UUAAAGGAAU GUAUGGACAA

6351 GUAUGGCCUG AACCUACCAA UGGUGACUUA UGUAAAAGAU GAGCUCAGGU

6401 CCAUAGAGAA GGUAGCGAAA GGAAAGUCUA GGCUGAUUGA GGCGUCCAGU

6451 UUGAAUGAUU CAGUGGCGAU GAGACAGACA UUUGGUAAUC UGUACAAAAC

6501 UUUCCACCUA AACCCAGGGG UUGUGACUGG UAGUGCUGUU GGGUGUGACC

6551 CAGACCUCUU UUGGAGCAAG AUACCAGUGA UGUUAGAUGG ACAUCUCAUA

6601 GCAUUUGAUU ACUCUGGGUA CGAUGCUAGC UUAAGCCCUG UCUGGUUUGC

6651 UUGCCUAAAA AUGUUACUUG AGAAGCUUGG AUACACGCAC AAAGAGACAA

6701 ACUACAUUGA CUACUUGUGC AACUCCCAUC ACCUGUACAG GGAUAAACAU

6751 UACUUUGUGA GGGGUGGCAU GCCCUCGGGA UGUUCUGGUA CCAGUAUUUU

6801 CAACUCAAUG AUUAACAAUA UCAUAAUUAG GACACUAAUG CUAAAAGUGU

6851 ACAAAGGGAU UGACUUGGAC CAAUUCAGGA UGAUCGCAUA UGGUGAUGAU

6901 GUGAUCGCAU CGUACCCAUG GCCUAUAGAU GCAUCUUUAC UCGCUGAAGC
6951 UGGUAAGGGU UACGGGCUGA UCAUGACACC AGCAGAUAAG GGAGAGUGCU
7001 UUAACGAAGU UACCUGGACC AACGCCACUU UCCUAAAGAG GUAUUUUAGA
7051 GCAGAUGAAC AGUACCCCUU CCUGGUGCAU CCUGUUAUGC CCAUGAAAGA
7101 CAUACACGAA UCAAUUAGAU GGACCAAGGA UCCAAAGAAC ACCCAAGAUC
7151 ACGUGCGCUC ACUGUGUCUA UUAGCUUGGC AUAACGGGGA GCACGAAUAU
7201 GAGGAGUUCA UCCGUAAAAU UAGAAGCGUC CCAGUCGGAC GUUGUUUGAC
7251 CCUCCCCGCG UUUUCAACUC UACGCAGGAA GUGGUUGGAC UCCUUUUAGA
7301 UUAGAGACAA UUUGAAAUAA UUUAGAUUGG CUUAACCCUA CUGUGCUAAC
7351 CGAACCAGAU AACGGUACAG UAGGGGUAAA UUCUCCGCAU UCGGUGCGG

Besonders bevorzugte rekombinante DNAs enthalten erfindungsgemäß die Teilsequenz von Nukleotid 532 bis 2041, Nukleotid 2289 bis 3600, oder Nukleotid 6059 bis 7130 der obengenannten Sequenz.

Ein weiterer Gegenstand der Erfindung sind Mikroorganismen, welche eine erfindungsgemäße rekombinante DNA enthalten. Besonders bevorzugt sind hierbei die transformierten E. coli Stämme VP4/2/3 (DSM 5558), VP1 und $3D^{Pol}$.

Ein weiterer Gegenstand der Erfindung ist ein Verfahren zur Herstellung eines erfindungsgemäßen Polypeptids, bei dem man das Polypeptid entweder aus einem der transformierten Mikroorganismen nach Aufschluß gewinnt oder eine erfindungsgemäße rekombinante DNA in geeignete Wirtszellen einbringt und das Polypeptid aus dem Kulturmedium, gegebenenfalls nach Aufschluß der Zellen, gewinnt. Vor allem bei Expression des Polypeptids in Eukaryonten erübrigt sich meist ein Zellaufschluß, da die Expressionsprodukte von den Zellen in das Kulturmedium abgegeben werden und hierdurch die Isolierung erleichtert wird.

Ein weiterer Gegenstand der Erfindung ist die Verwendung eines oder mehrerer der erfindungsgemäßen Polypeptide, vorzugsweise einem der Polypeptide VP4/2/3, VP1 oder der Polymerase zur Erzeugung von gruppenspezifischen polyklonalen oder monoklonalen Antikörpern gegen Enteroviren. Hierzu müssen jedoch nicht unbedingt die vollständigen Polypeptide verwendet werden, solange mindestens eine der entsprechenden antigenen Determinanten auf einem unvollständigen Polypeptid vorhanden ist.

Die Möglichkeit, Infektionen durch einen Virus der gesamten Familie der Enteroviren spezifisch nachzuweisen, wird durch einen weiteren Gegenstand der Erfindung, nämlich den gruppenspezifischen immunologischen Nachweis von Enteroviren bzw. Antikörpern im Blut oder Serum von zu untersuchenden Patienten ermöglicht, bei dem man in einem an sich bekannten immunologischen Test, vorzugsweise einem ELISA, mindestens eines der erfindungsgemäßen Polypeptide als Antigen oder gegen diese Polypeptide erzeugte Antikörper einsetzt. Bei der erfindungsgemäßen gruppenspezifischen Nachweismethode sind alle dem Fachmann geläufigen immunologischen Testmethoden anwendbar, bei denen im Blut oder Serum von Patienten Antigene oder Antikörper nachgewiesen werden sollen. Beim Nachweis von Antigenen im Blut oder Serum werden Antikörper gegen die erfindungsgemäßen Polypeptide eingesetzt, die dann das im Blut oder Serum vorhandene Antigen binden. Zum Nachweis von Antikörpern im Blut oder Serum von Patienten können die Polypeptide selbst sowie die mit den Polypeptiden generierten Antikörper eingesetzt werden. Der Nachweis über markierte Antigene oder Antikörper und die praktische Durchführung des Tests, z.B. mit wandgebundenen Antikörpern, Antigenen etc., wird nach an sich bekannten Methoden durchgeführt.

Erfindungsgemäß wird es durch die Auffindung der erfindungsgemäßen Polypeptide, welche für alle über 70 Serotypen von Enteroviren spezifische antigene Determinanten enthalten, erstmals ermöglicht, einen gruppenspezifischen Nachweis gegen die gesamte Familie der Enteroviren zu führen. Dies ermöglicht die rasche und sichere Serodiagnostik von Patienten mit klinischem Verdacht auf Enterovirus-Infektionen, z.B. Verdacht auf Myokarditis, Myositis, Meningitis, Encephalitis, Pancreatitis oder auch postvirales Fatigue-Syndrom.

Fig. 1 zeigt hierbei den Aufbau eines direkten ELISA,

Fig. 2 zeigt den eines Sandwich-ELISA.

Die folgenden Beispiele erläutern in Verbindung mit den Abbildungen die Erfindung weiter.

**Beispiel 1**

Herstellung der Polypeptide

Zur Expression von Coxsackie-Virus B3 (CVB3) wurden die transformierten E. coli Stämme VP4/2/3, VP1, $3D^{Pol}$ verwendet. Jeweils 100 ml einer frischen Übernachtkultur wurden in 350 ml selektives LB-Medium (LB-Medium supplementiert mit 100 µg/ml Ampicillin) überimpft und bis zu einer optischen Dichte bei 620 nm von 1,2 bis 2,0 wachsen gelassen. Danach wurde die Temperatur auf 42° C erhöht, indem 450 ml 56° C warmes Medium langsam und unter ständigem Schwenken zugegeben wurden. Die Expressionstemperatur wurde für eine Stunde beibehalten. Anschließend wurden die Bakterien bei 3500 g (40 Minuten, 4° C) pelletiert. Die Pellets wurden zur Lyse der Bakterien in $PBS^-$ (0,137 mmol/1 NaCl, 3 mmol/l KCl, 6 mmol/l $Na_2HPO_4$, 1 mmol/l $KH_2PO_4$), pH 7,2/1 % SDS (Natriumdodecylsulfat) resuspendiert und 10 Minuten im siedenden Wasserbad erhitzt. Die so erhaltene klare, proteinhaltige Lösung konnte direkt auf Polyacrylamidgele aufgetragen werden. Für den Einsatz im ELISA war die SDS-Konzentration jedoch zu hoch, so daß SDS mit einem zweifachen molaren Überschuß an KCl ausgefällt wurde.

**Beispiel 2**

A) Isolation rekombinanter Proteine durch gelelektrophoretische Methoden

Das Bakterienzellysat wurde zur Auftrennung der Proteine auf ein vertikales Polyacrylamidgel (5%iges Sammelgel/l3%iges Trenngel) aufgetragen und im elektrischen Feld aufgetrennt (Laemmli., 1970, Nature 227, S. 680-685). Nach der Elektrophorese wurden die Proteinbanden durch eine Färbung mit Coomassie Blue sichtbar gemacht und anschließend distinkte virale Proteine präparativ rückisoliert. Dazu wurden die entsprechenden Banden aus dem Gel ausgeschnitten und die Proteine aus dem Acrylamid im elektrischen Feld eluiert (Hunkapiller et al., 1983, Meth. Enzymol. 91, S. 227-236). Die Reinheit der gewonnenen Proteinproben wurde, nach Proteinbestimmung (W. Schaffner und C. Weissmann, (1973), A rapid, sensitive and specific method for the determination of protein in dilute solution. Anal. Biochem. 56, 502-514) in einer analytischen, vertikalen Polyacrylamid-Gelelektrophorese überprüft.

B) Isolation und Reinigung viraler Antigene durch Affinitäts-Chromatographie

Zur Minimierung unspezifischer Reaktionen im ELISA wurden die viralen Antigene vor Gebrauch über eine Affinitätssäule gereinigt. Dazu wurden die polyklonalen Antiseren, (entweder aVP1 oder aVP4/2/3 oder $a3D^{Pol}$) an Bromcyan aktivierte Sepharose gekoppelt. Die viralen Antigene enthalten in einem Lysat CVB3 (Coxsackie-Virus B3) infizierter Verozellen, wurden in Bindungspuffer (20 mM Phosphatpuffer, pH 8,0) aufgenommen und mit einer Flußgeschwindigkeit von 0,05 ml/min über die entsprechenden Säulen aufgetrennt. Die adsorbierten Antigene (VP1 oder VP4/2/3 oder $3D^{Pol}$) wurden anschließend mit derselben Flußgeschwindigkeit und einem Eluierungspuffer (100 mM Glycin, pH 2,7) von den gebundenen Antikörpern getrennt und in einem Eppendorfgefäß mit 0,5 M Carbonatpuffer aufgefangen. Zur Stabilisierung der Proben wurden diese anschließend über PD-10 Säulen (Pharmacia) in $PBS^-$umgepuffert.

**Beispiel 3**

Reinigung der rekombinanten Proteine über eine Hydroxylapatit-Säule

Proteinproben, die zur Immunisierung verwendet werden, sollten keine zu hohe Konzentration an Detergentien, wie z.B. SDS oder NP40, enthalten, da dies für das Versuchstier tödlich sein kann. Deshalb wurden die Proben mit Hilfe einer Hydroxylapatit-Säule (G. Bernadi, Meth. Enzymol. 22 (1971) 325-339) von diesen Detergentien weitgehend befreit. Die Bindung der Proteine an die Matrix erfolgte in einem 0,01 mol/l Natriumphosphat-Puffer (pH 6,8). Im zweiten Schritt wurde das Detergens herausgewaschen, in dem 4 bis 5 Säulenvolumen desselben Puffers über die Säule gegeben wurden. Im letzten Schritt wurde dann das Protein mit einem 0,5 mol/l Na-Phosphat-Puffer (pH 6,8) eluiert.

**Beispiel 4**

Generierung monoklonaler Antikörper und polyklonaler Antiseren

\- Balb/c-Mäuse -

Zur Generierung monoklonaler Antikörper wurden ca. 6 Wochen alte weibliche Balb/c-Mäuse nach folgendem Immunisierungsschema mit CVB3 infiziertem Verozell-Lysat immunisiert.

| Tag | | Proteindosis (ug) gelöst in: | CFA | IFA | $PBS^-$ |
|---|---|---|---|---|---|
| 1 | Priming | 150 ug | * | | |
| 21 | Auffrischung | 50 ug | | * | |
| 35 | Auffrischung | 50 ug | | * | |
| 50 | Auffrischung | 50 ug | | | * |
| 51 | Boost | 20 ug | | | * |
| 52 | Boost | 20 ug | | | * |
| 53 | Boost | 20 ug | | | * |

CFA = komplettes Freund'sches Adjuvans
IFA = inkomplettes Freund'sches Adjuvans
PBS = phosphatgepufferte Salzlösung

Für die einzelnen Immunisierungsschritte wurde die entsprechende Proteinmenge in den jeweiligen Zusätzen emulgiert, 2 Stunden bei Raumtemperatur inkubiert und danach den Mäusen i.p. injiziert. Zur Überprüfung der Immuinantwort wurde nach der ersten und zweiten Auffrischung den Mäusen Blut aus der Schwanzvene entnommen und der Antikörpertiter des Serums gegen die rekombinanten CVB3-Proteine (VP1 oder VP4/2/3 oder $3D^{pol}$) im ELISA (siehe Beispiel 5A) bestimmt. Die Fusion der Immunglobulin-produzierenden Milzzellen der nach obigem Schema immunisierten Mäuse und der Maus-Myelom Permanentzellinie X63/Ag8.653 wurde nach der von G. Köhler und C. Milstein 1975 beschriebenen Methode durchgeführt (Nature 256, 495-497). Es schloß sich eine 14tägige Selektion durch HAT-Medium an, die sicherstellt, daß nur durch die Fusion entstandene Zellklone überleben. Die so erhaltenen Zellklone wurden nach der "Limiting Dilution" zweimal subkloniert. Die Zellklone, die spezifische Antikörper gegen CVB3-Proteine produzieren, wurden weiter kultiviert und einmal wöchentlich das Medium zu 2/3 abgenommen und durch frisches Medium ersetzt. Der abgenommene Zellüberstand wurde 5 Minuten bei 700 g zentrifugiert und der klare Überstand bei -20 °C eingefroren. In diesem Überstand sind ca. 5 bis 20 ug/ml monoklonale Antikörper enthalten, so daß sie 1:10 verdünnt im ELISA eingesetzt werden können.

- Kaninchen -

Zur Generierung der polyklonalen Antiseren wurden drei verschiedene, je sechs Monate alte weibliche Neuseeland-Kaninchen mit in E. coli exprimierten, aufgereinigten CVB3-Proteinen (entweder VP1 oder VP4/2/3 oder $3D^{pol}$) nach folgendem Schema immunisiert:

| Tag | | Proteindosis (ug) in : | CFA | IFA | PBS |
|---|---|---|---|---|---|
| 1 | Priming | 150 ug | * | | |
| 13 | Auffrischung | 75 ug | | * | |
| 36 | Auffrischung | 75 ug | | * | |
| 64 | Auffrischung | 75 ug | | | * |

alle 3 Monate eine weitere Auffrischung mit 75 ug Protein in $PBS^-$.

Vor jeder Auffrischung wurde dem Kaninchen 5 ml Blut aus der Ohrvene entnommen und der jeweilige anti-CVB3-Protein-Antikörpertiter im ELISA bestimmt. 64 Tage nach der Erstimmunisierung war der Antikörpertiter so weit gestiegen, daß dem Kaninchen in vierwöchigen Abständen 20 ml Blut aus der Ohrvene abgenommen werden konnte. Nach erfolgter Blutgerinnung bei Raumtemperatur wurde das Serum durch eine 10minutige Zentrifugation bei 300 g gewonnen, anschließend aliquotiert und bei -20° C aufbewahrt.

**Beispiel 5**

ELISA (Enzyme Linked Immuno Sorbent Assay)

Im ELISA lassen sich Antigen-Antikörper-Wechselwirkungen messen. Das Prinzip beruht darauf, daß entweder das Antigen an die Festphase gebunden, angeboten wird und dann der Antikörpertiter bzw. die Existenz von Antikörpern in einem Serum gemessen wird, oder die Antikörper an die Festphase gebunden, angeboten werden und somit die Konzentration von Antigenen in einem Serum gemessen werden können.

Allgemein differenziert man zwischen zwei ELISA-Formen, dem direkten ELISA (siehe Fig. 1), hierbei wird das Substrat (entweder Antigen oder Antikörper) direkt an die Festphase gebunden und dem Sandwich ELISA (siehe Figur 2). Bei diesem Prinzip wird das Antigen von einem Antikörper gebunden frei im Raum und in nativer Form präsentiert. Dies führt zu einer erheblichen Sensitivitätssteigerung des Testsystems (Moudalal et al., 1984, J. Immunol. Meth. 68, S. 35 - 43).

Lösungen:

Beschichtungspuffer: 100 mM Carbonatpuffer, pH 9,6
Absättigungslösung: 0,5 % BSA (Rinderserumalbumin) in $PBS^-$
Waschlösung: 1,5 M NaCl 20 % Tween 20
Substratlösung:
a) 10 % Diethanolaminpuffer: 97 ml 1 M Diethanolamin 100 mg $MgCl_2$x 6 $H_2O$ mit 1M H Cl pH 9,8 einstellen ad 1000 ml $H_2O$-bidest.

b) PNPP(p-Nitro-phenyl-phosphat) in Diethanolaminpuffer lösen mit einer Konzentration von 1 mg/ml
Stopp-Lösung: 3 M NaOH.

A) Direkter ELISA

Nachweis von enteroviralen Antikörpern in human Seren (Figur 1)

Eines der viralen Antigene (VP1 oder VP4/2/3 oder $3D^{pol}$), gewonnen und gereinigt aus infizierten Vero-Zellysaten (siehe Beispiel 2B) wurde in einer Konzentration von 300 ng/100 µl/Well (Vertiefung einer Plastik-Mikrotiterplatte) in Beschichtungspuffer verdünnt und bei 4° C inkubiert. Es folgt ein einmaliges Waschen mit der Waschlösung. Zur Absättigung freier Bindungsstellen der Plastikoberfläche wurden 200 µl Absättigungslösung/Well für eine Stunde bei Raumtemperatur inkubiert. Nach zweimaligem Waschen mit Waschlösung wurde das zu untersuchende Patientenserum in einer 1:10 Verdünnung (in $PBS^-$) in einem Volumen von 100 µl für 2 Stunden bei 37° C inkubiert. Es folgt ein dreimaliges Waschen mit der Waschlösung. Zum Nachweis der an CVB-Antigen gebundenen humanen Antikörper wird ein anti-human Immunglobulin-Konjugat mit der alkalischen Phosphatase (AP-Konjugat) in einem Volumen von 100 µl für eine Stunde bei 37° C inkubiert. Nach dreimaligem Waschen mit Waschlösung wurden 100 µl PNPP-Substratlösung für 30 bis 60 Minuten bei 37° C im Dunklen inkubiert, anschließend wurde die Enzymreaktion durch Zugabe von 100 µl Stopp-Lösung gestoppt und die Extinktion bei einer optischen Dichte von 405 nm ($OD_{405nm}$) gemessen.

Nachweis von enteroviralem Antigen in human Seren (Figur 1)

Alle Waschschritte erfolgen analog dem oben beschriebenen Schemata. Die Patientenseren wurden 1:10 in Beschichtungspuffer verdünnt und für 18 Stunden bei 4° C inkubiert. Zur Absättigung freier Bindungsstellen der Plastikoberfläche wurden 200 µl Absättigungslösung/Well für 2 Stunden bei Raumtemperatur inkubiert. Es folgt eine Inkubation mit Enterovirusspezifischen polyklonalen Antiseren (aVP1 oder aVP4/2/3 oder $a3D^{pol}$) 1:500 verdünnt in $PBS^-$ für 2 Stunden bei 37° C. Der Antigen-Antikörper-Komplex wurde durch eine einstündige Inkubation bei 37° C mit einem AP-Konjugat anti-Kaninchen-Immunglobulin in einem Volumen von 100 µl nachgewiesen. Zuletzt wurden 100 µ1 PNPP-Substratlösung für 30 bis 60 Minuten bei 37° C im Dunklen inkubiert, die Enzymreaktion durch Zugabe von 100 µl Stopp-Lösung gestoppt und die Extinktion bei einer $0D_{405nm}$ gemessen.

B) Sandwich ELISA (Figur 2)

Als erste Komponente wurde eines der Affinitäts-gereinigten polyklonalen und Enterovirus-spezifischen Antiseren (aVP1 oder aVP4/2/3 oder $a3D^{pol}$) in einer Konzentration von 300 ng/100 µl/Well an die Plastikoberfläche gebunden (18 Stunden bei 4° C). Nach zweimaligem Waschen mit Waschlösung wurde das entsprechende aufgereinigte Antigen aus infizierten Verozell-Lysaten (siehe Beispiel 2B) (VP1 oder VP4/2/3 oder $3D^{Pol}$) in einer Konzentration von 300 ng/100 µl/Well für 3 Stunden bei 37° C vorgelegt. Es schließen sich zwei Waschschritte an, bevor das 1:10 in PBS verdünnte Patientenserum für eine Stunde bei 37° C inkubiert wurde. Nachdem dreimal gewaschen wurde, wurde das "Immun-Sandwich" bestehend aus polyklonalem Antikörper (Festphasenkomponente), Antigen und humanem Antikörper mit einem AP-Konjugat anti-human Immunglobulin nachgewiesen, durch eine einstündige Inkubation der entsprechenden Konjugatlösung. Vor der Substratreaktion wurde wiederum dreimal gewaschen, 100 µl Substratlösung zugegeben und nach 30 bis 60 Minuten Inkubation bei 37° C im Dunklen die Enzymreaktion mit 100 µl Stopp-Lösung abgebrochen. Zuletzt wurde wiederum die Extinktion bei einer $OD_{405nm}$ gemessen.

**Beispiel 6**

Immunoblot (Western-Blot)

Die durch SDS-Polyacrylamid-Gelelektrophorese (PAGE) aufgetrennten, affinitätschromatographisch gereinigten viralen Antigene (VP1 oder VP4/2/3 oder $3D^{Pol}$) wurden mittels Elektro-Blotting (Semi-dry) auf Nitrocellulose transferiert. Der Nachweis dieser Antigene erfolgt durch eine spezifische Antikörperreaktion und nachfolgender enzymatischer Farbreaktion (Towbin et al., 1979, Proc. Natl. Acad. Sci. 76, S. 4350-4354), wodurch die humanen Antikörper (aus Patientenserum) spezifisch charakterisiert werden.

Lösungen:

| | |
|---|---|
| Lösung I : | 25 mM Tris/HCl, pH 8,0 |
| | 76 mM NaCl |
| | 0,5 % Gelatine |
| | 2,5 % BSA |
| | 0,003 % NP40 |
| Lösung II: | 50 mM Tris/HCl, pH 8,0 |
| | 15 mM NaCl |
| | 0,25 % Gelatine |
| | 0,1 % BSA |
| | 0,025 % NP40 |
| Antikörperlösung: | 50 mM Tris/HCl, pH 8,0 |
| | 15 mM NaCl |
| | 0,25 % Gelatine |
| | 3,0 % BSA |
| | 0,025 % NP40 |

Substratlösung: 100 mM Tris/HCl, pH 9,5
100 mm NaCl
5 mM $MgCl_2$
400 uM NBT in 70% Dimethylformamid
400 uM BCIP in 100 % Dimethylformamid

NBT = Nitro-Blue-tetrazolium
BCIP = 5 Bromo- 4 Chloro- 3 Indolyl-Phosphat

Nach erfolgter SDS-PAGE der viralen Antigene VPO1, VP4/2/3, $3D^{pol}$ wurde auf der Kathode der Semi-Dry Apparatur das Polyacrylamidgel mit dem Nitrocellulosefilter (NC-Filter) als Sandwich zwischen jeweils drei Schichten Whatman 3-MM-Filterpapier aufgebaut. Die obere Graphitplatte ( = Anode) wurde aufgesetzt und der Transfer mit einer konstanten Stromstärke von 150 mA über eine Zeitspanne von 90 Minuten durchgeführt.

Als Vorbereitung der Immunreaktion wurden die noch freien Proteinbindungsstellen der NC durch zweimal 10 Minuten Schwenken in Lösung I abgesättigt. Das Patientenserum wurde 1:20 in Antikörperlösung verdünnt und eine Stunde mit dem Filter inkubiert. Zum Entfernen der nicht- oder unspezifisch gebundenen Antikörper wurde die NC dreimal 15 Minuten in Lösung II geschwenkt. Der zweite Antikörper, ein AP-anti-human-Immunglobulin-Konjugat 1:500 verdünnt in Antikörperlösung, wurde ebenfalls für eine Stunde mit dem NC-Filter inkubiert. Es folgte ein dreimaliges Waschen mit Lösung II für jeweils 15 Minuten. Vor der Farbreaktion wurde der Filter zwischen 3-MM-Whatmanpapier getrocknet. Es schloß sich eine Inkubation der NC in der Substratlösung für 5 bis 15 Minuten an, danach wurde die Enzymreaktion durch sechsmal Waschen des Filters mit destilliertem Wasser gestoppt. Alle Inkubationsschritte wurden bei Raumtemperatur ausgeführt. Durch die Charakterisierung der humanen Antiseren im Western-Blot konnte gezeigt werden, daß tatsächlich ein entsprechendes Protein mit dem richtigen Molekulargewicht vom Antiserum erkannt wird.

## Ansprüche

1. Polypeptid, **gekennzeichnet durch** die folgende Aminosäuresequenz

MGAQVSTQ KTGAHETRLN ASGNSIIHYT NINYYKDAAS NSANRQDFTQ
DPGKFTEPVK DIMIKSLPAL NSPTVEECGY SDRARSITLG NSTITTQECA
NVVVGYGVWP DYLKDSEATA EDQPTQPDVA TCRFYTLDSV QWQKTSPGWW
WKLPDALSNL GLFGQNMQYH YLGRTGYTVH VQCNASKFHQ GCLLVVCVPE
AEMGCATLDN TPSSAELLGG DTAKEFADKP VASGSNKLVQ RVVYNAGMGV
GVGNLTIFPH QWINLRTNNS ATIVMPYTNS VPMDNMFRHN NVTLMVIPFV
PLDYCPGSTT YVPITVTIAP MCAEYNGLRL AGHQGLPTMN TPGSCQFLTS
DDFQSPSAMP QYDVTPEMRI PGEVKNLMEI AEVDSVVPVQ NVGEKVNSME
AYQIPVRSNE GSGTQVFGFP LQPGYSSVFS RTLLGEILNY YTHWSGSIKL
TFMFCGSAMA TGKFLLAYSP PGAGAPTKRV DAMLGTHVIW DVGLQSSCVL
CIPWISQTHY RFVASDEYTA GGFITCWYQT NIVVPADAQS SCYIMCFVSA
CNDFSVRLLK DTPFISQQNF FQGPVEDAIT AAIGRVADTV GTGPTNSEAI
PALTAAETGH TSQVVPGDTM QTRHVKNYHS RSESTIENFL CRSACVYFTE
YKNSGAKRYA EWVLTPRQAA QLRRKLEFFT YVRFDLELTF VITSTQQPST
TQNQDAQILT HQIMYVPPGG PVPDKVDSYV WQTSTNPSVF WTEGNAPPRM
SIPFLSIGNA YSNFYDGWSE FSRNGVYGIN TLNNMGTLYA RHVNAGSTGP
IKSTIRIYFK PKHVKAWIPR PPRLCQYEKA KNVNFQPSGV TTTRQSITTM
TNTGAFGQQS GAVYVGNYRV VNRHLATSAD WQNCVWESYN RDLLVSTTTA
HGCDIIARCQ CTTGVYFCAS KNKHYPISFE GPGLVEVQES EYYPRRYQSH
VLLAAGFSEP GDCGGILRCE HGVIGIVTMG GEGVVGFADI RDLLWLEDDA
MEQGVKDYVE QLGNAFGSGF TNQICEQVNL LKESLVGQDS ILEKSLKALV
KIISALVIVV RNHDDLITVT ATLALIGCTS SPWRWLKQKV SQYYGIPMAE
RQNNSWLKKF TEMTNACKGM EWIAVKIQKF IEWLKVKILP EVREKHEFLN
RLKQLPLLES QIATIEQSAP SQSDQEQLFS NVQYFAHYCR KYAPLYAAEA
KRVFSLEKKM SNYIQFKSKC RIEPVCLLLH GSPGAGKSVA TNLIGRSLAE
KLNSSVYSLP PDPDHFDGYK QQAVVIMDDL CQNPDGKDVS LFCQMVSSVD
FVPPMAALEE KGILFTSPFV LASTNAGSIN APTVSDSRAL ARRFHFDMNI
EVISMYSQNG KINMPMSVKT CDDECCPVNF KKCCPLVCGK AIQFIDRRTQ
VRYSLDMLVT EMFREYNHRH SVGTTLEALF QGPPVYREIK ISVAPETPPP

PAIADLLKSV DSEAVREYCK EKGWLVPEIN STLQIEKHVS RAFICLQALT
TFVSVAGIIY IIYKLFAGFQ GAYTGVPNQK PRVPTLRQAK VQGPAFEFAV
AMMKRNSSTV KTEYGEFTML GIYDRWAVLP RHAKPGPTIL MNDQEVGVLD
AKELVDKDGT NLELTLLKLN RNEKFRDIRG FLAKEEVEVN EAVLAINTSK
FPNMYIPVGQ VTEYGFLNLG GTPTKRMLMY NFPTRAGQCG GVLMSTGKVL
GIHVGGNGHQ GFSAALLKHY FNDEQGEIEF IESSKDAGFP VINTPSKTKL
EPSVFHQVFE GNKEPAVLRS GDPRLKANFE EAIFSKYIGN VNTHVDEYML
EAVDHYAGQL ATLDISTEPM KLEDAVYGTE GLEALDLTTS AGYPYVALGI
KKRDILSKKT KDLTKLKECM DKYGLNLPMV TYVKDELRSI EKVAKGKSRL
IEASSLNDSV AMRQTFGNLY KTFHLNPGVV TGSAVGCDPD LFWSKIPVML
DGHLIAFDYS GYDASLSPVW FACLKMLLEK LGYTHKETNY IDYLCNSHHL
YRDKHYFVRG GMPSGCSGTS IFNSMINNII IRTLMLKVYK GIDLDQFRMI
AYGDDVIASY PWPIDASLLA EAGKGYGLIM TPADKGECFN EVTWTNATFL
KRYFRADEQY PFLVHPVMPM KDIHESIRWT KDPKNTQDHV RSLCLLAWHN
GEHEYEEFIR KIRSVPVGRC LTLPAFSTLR RKWLDSF

2. Polypeptid, **dadurch gekennzeichnet,** daß es die im folgenden angegebene Teilsequenz der Sequenz von Anspruch 1 enthält (VP4/2/3).

AQVSTQ KTGAHETRLN ASGNSIIHYT NINYYKDAAS NSANRQDFTQ
DPGKFTEPVK DIMIKSLPAL NSPTVEECGY SDRARSITLG NSTITTQECA
NVVVGYGVWP DYLKDSEATA EDQPTQPDVA TCRFYTLDSV QWQKTSPGWW
WKLPDALSNL GLFGQNMQYH YLGRTGYTVH VQCNASKFHQ GCLLVVCVPE
AEMGCATLDN TPSSAELLGG DTAKEFADKP VASGSNKLVQ RVVYNAGMGV
GVGNLTIFPH QWINLRTNNS ATIVMPYTNS VPMDNMFRHN NVTLMVIPFV
PLDYCPGSTT YVPITVTIAP MCAEYNGLRL AGHQGLPTMN TPGSCQFLTS
DDFQSPSAMP QYDVTPEMRI PGEVKNLMEI AEVDSVVPVQ NVGEKVNSME
AYQIPVRSNE GSGTQVFGFP LQPGYSSVFS RTLLGEILNY YTHWSGSI

3. Polypeptid, **dadurch gekennzeichnet,** daß es die im folgenden angegebene Teilsequenz der Sequenz von Anspruch 1 enthält (VP1).

SDEYTA GGFITCWYQT NIVVPADAQS SCYIMCFVSA
CNDFSVRLLK DTPFISQQNF FQGPVEDAIT AAIGRVADTV GTGPTNSEAI
PALTAAETGH TSQVVPGDTM QTRHVKNYHS RSESTIENFL CRSACVYFTE
YKNSGAKRYA EWVLTPRQAA QLRRKLEFFT YVRFDLELTF VITSTQQPST
TQNQDAQILT HQIMYVPPGG PVPDKVDSYV WQTSTNPSVF WTEGNAPPRM
STPFLSIGNA YSNFYDGWSE FSRNGVYGIN TLNNMGTLYA RHVNAGSTGP
IKSTIRIYFK PKHVKAWIPR PPRLCQYEKA KNVNFQPSGV TTTRQSITTM
TNTGAFGQQS GAVYVGNYRV VNRHLATSAD WQNCVWESYN RDLLVSTTTA
HGCDIIARCQ CTTGVYFCAS KNKHYPISFE GPGLVEVQES EYYPRRYQSH

VL

4. Polypeptid, **dadurch gekennzeichnet,** daß es die im folgenden angegebene Teilsequenz der Sequenz von Anspruch 1 enthält (3D-Pol).

RS GDPRLKANFE EAIFSKYIGN VNTHVDEYML
EAVDHYAGQL ATLDISTEPM KLEDAVYGTE GLEALDLTTS AGYPYVALGI
KKRDILSKKT KDLTKLKECM DKYGLNLPMV TYVKDELRSI EKVAKGKSRL
IEASSLNDSV AMRQTFGNLY KTFHLNPGVV TGSAVGCDPD LFWSKIPVML
DGHLIAFDYS GYDASLSPVW FACLKMLLEK LGYTHKETNY IDYLCNSHHL
YRDKHYFVRG GMPSGCSGTS IFNSMINNII IRTLMLKVYK GIDLDQFRMI
AYGDDVIASY PWPIDASLLA EAGKGYGLIM TPADKGECFN EVTWTNATFL
KRYFRADEQY PFLVHPVMPM KDIHESIRW

5. Polypeptid, **dadurch gekennzeichnet,** daß es mindestens eine der auf den Polypeptiden nach einem der Ansprüche 1 bis 4 enthaltenen antigenen Determinanten enthält.

6. Rekombinante DNA, **dadurch gekennzeichnet,** daß sie eine DNA-Sequenz, die für ein Polypeptid nach einem der Ansprüche 1 bis 5 kodiert, einligiert in einen Espressionsvektor enthält.

7. Rekombinante DNA nach Anspruch 6, **dadurch gekennzeichnet,** daß sie die folgende Sequenz enthält:

```
  1 UUAAAACAGC CUGUGGGUUG AUCCCACCCA CAGGCCCAUU GGGCGCUAGC
 51 ACUCUGGUAU CACGGUACCU UUGUGCGCCU GUUUUAUACC CCCUCCCCCA
101 ACUGUAACUU AGAAGUAACA CACACCGAUC AACAGUCAGC GUGGCACACC
151 AGCCACGUUU UGAUCAAGCA CUUCUGUUAC CCCGGACUGA GUAUCAAUAG
201 ACUGCUCACG CGGUUGAAGG AGAAAGCGUU CGUUAUCCGG CCAACUACUU
251 CGAAAAACCU AGUAACACCG UGGAAGUUGC AGAGUGUUUC GCUCAGCACU
301 ACCCCAGUGU AGAUCAGGUC GAUGAGUCAC CGCAUUCCCC ACGGGCGACC
351 GUGGCGGUGG CUGCGUUGGC GGCCUGCCCA UGGGGAAACC CAUGGGACGC
401 UCUAAUACAG ACAUGGUGCG AAGAGUCUAU UGAGCUAGUU GGUAGUCCUC
451 CGGCCCCUGA AUGCGGCUAA UCCUAACUGC GGAGCACACA CCCUCAAGCC
501 AGAGGGCAGU GUGUCGUAAC GGGCAACUCU GCAGCGGAAC CGACUACUUU
551 GGGUGUCCGU GUUUCAUUUU AUUCCUAUAC UGGCUGCUUA UGGUGACAAU
601 UGAGAGAUCG UUACCAUAUA GCUAUUGGAU UGGCCAUCCG GUGACUAAUA
651 GAGCUAUUAU AUAUCCCUUU GUUGGGUUUA UACCACUUAG CUUGAAAGAG
701 GUUAAAACAU UACAAUUCAU UGUUAAGUUG AAUACAGCAA AAUGGGAGCU
751 CAAGUAUCAA CGCAAAAGAC UGGGGCACAU GAGACCAGGC UGAAUGCUAG
801 CGGCAAUUCC AUCAUUCACU ACACAAAUAU UAAUUAUUAC AAGGAUGCCG
851 CAUCCAACUC AGCCAAUCGG CAGGAUUUCA CUCAAGACCC GGGCAAGUUC
901 ACAGAACCAG UGAAAGAUAU CAUGAUUAAA UCACUACCAG CUCUCAACUC
951 CCCCACAGUA GAGGAGUGCG GAUACAGUGA CAGGGCGAGA UCAAUCACAU
```

1001 UAGGUAACUC CACCAUAACG ACUCAGGAAU GCGCCAACGU GGUGGUGGGC
1051 UAUGGAGUAU GGCCAGAUUA UCUAAAGGAU AGUGAGGCAA CAGCAGAGGA
1101 CCAACCGACC CAACCAGACG UUGCCACAUG UAGGUUCUAU ACCCUUGACU
1151 CUGUGCAAUG GCAGAAAACC UCACCAGGAU GGUGGUGGAA GCUGCCCGAU
1201 GCUUUGUCGA ACUUAGGACU GUUUGGGCAG AACAUGCAGU ACCACUACUU
1251 AGGCCGAACU GGGUAUACCG UACAUGUGCA GUGCAAUGCA UCUAAGUUCC
1301 ACCAAGGAUG CUUGCUAGUA GUGUGUGUAC CGGAAGCUGA GAUGGGUUGC
1351 GCAACGCUAG ACAACACCCC AUCCAGUGCA GAAUUGCUGG GGGGCGAUAC
1401 GGCAAAGGAG UUUGCGGACA AACCGGUCGC AUCCGGGUCC AACAAGUUGG
1451 UACAGAGGGU GGUGUAUAAU GCAGGCAUGG GGGUGGGUGU UGGAAACCUC
1501 ACCAUUUUCC CCCACCAAUG GAUCAACCUA CGCACCAAUA AUAGUGCUAC
1551 AAUUGUGAUG CCAUACACCA ACAGUGUACC UAUGGAUAAC AUGUUUAGGC
1601 AUAACAACGU CACCCUAAUG GUUAUCCCAU UUGUACCGCU AGAUUACUGC
1651 CCUGGGUCCA CCACGUACGU CCCAAUUACG GUCACGAUAG CCCCAAUGUG
1701 UGCCGAGUAC AAUGGGUUAC GUUUAGCAGG GCACCAGGGC UUACCAACCA
1751 UGAAUACUCC GGGGAGCUGU CAAUUUCUGA CAUCAGACGA CUUCCAAUCA
1801 CCAUCCGCCA UGCCGCAAUA UGACGUCACA CCAGAGAUGA GGAUACCUGG
1851 UGAGGUGAAA AACUUGAUGG AAAUAGCUGA GGUUGACUCA GUUGUCCCAG
1901 UCCAAAAUGU UGGAGAGAAG GUCAACUCUA UGGAAGCAUA CCAGAUACCU
1951 GUGAGAUCCA ACGAAGGAUC UGGAACGCAA GUAUUCGGCU UUCCACUGCA
2001 ACCAGGGUAC UCGAGUGUUU UUAGUCGGAC GCUCCUAGGA GAGAUCUUGA
2051 ACUAUUAUAC ACAUUGGUCA GGCAGCAUAA AGCUUACGUU UAUGUUCUGU
2101 GGUUCGGCCA UGGCUACUGG AAAAUUCCUU UUGGCAUACU CACCACCAGG
2151 UGCUGGAGCU CCUACAAAAA GGGUUGAUGC UAUGCUUGGU ACUCAUGUAA
2201 UUUGGGACGU GGGGCUACAA UCAAGUUGCG UGCUGUGUAU ACCCUGGAUA
2251 AGCCAAACAC ACUACCGGUU UGUUGCUUCA GAUGAGUAUA CCGCAGGGGG
2301 UUUUAUUACG UGCUGGUAUC AAACAAACAU AGUGGUCCCA GCGGAUGCCC
2351 AAAGCUCCUG UUACAUCAUG UGUUUCGUGU CAGCAUGCAA UGACUUCUCU
2401 GUCAGGCUAU UGAAGGACAC UCCUUUCAUU UCGCAGCAAA ACUUUUUCCA
2451 GGGCCCAGUG GAAGACGCGA UAACAGCCGC UAUAGGGAGA GUUGCGGAUA
2501 CCGUGGGUAC AGGGCCAACC AACUCAGAAG CUAUACCAGC ACUCACUGCU
2551 GCUGAGACGG GUCACACGUC ACAAGUAGUG CCGGGUGACA CUAUGCAGAC
2601 ACGCCACGUU AAGAACUACC AUUCAAGGUC CGAGUCAACC AUAGAGAACU
2651 UCCUAUGUAG GUCAGCAUGC GUGUACUUUA CGGAGUAUAA AAACUCAGGU

2701 GCCAAGCGGU AUGCUGAAUG GGUAUUAACA CCACGACAAG CAGCACAACU
2751 UAGGAGAAAG CUAGAAUUCU UUACCUACGU CCGGUUCGAC CUGGAGCUGA
2801 CGUUUGUCAU AACAAGUACU CAACAGCCCU CAACCACACA GAACCAAGAU
2851 GCACAGAUCC UAACACACCA AAUUAUGUAU GUACCACCAG GUGGACCUGU
2901 ACCAGAUAAA GUUGAUUCAU ACGUGUGGCA AACAUCUACG AAUCCCAGUG
2951 UGUUUUGGAC CGAGGGAAAC GCCCCGCCGC GCAUGUCCAU ACCGUUUUUG
3001 AGCAUUGGCA ACGCCUAUUC AAAUUUCUAU GACGGAUGGU CUGAAUUUUC
3051 CAGGAACGGA GUUUACGGCA UCAACACGCU AAACAACAUG GGCACGCUAU
3101 AUGCAAGACA UGUCAACGCU GGAAGCACGG GUCCAAUAAA AAGCACCAUU
3151 AGAAUCUACU UCAAACCGAA GCAUGUCAAA GCGUGGAUAC CUAGACCACC
3201 UAGACUCUGC CAAUACGAGA AGGCAAAGAA CGUGAACUUC CAACCCAGCG
3251 GAGUUACCAC UACUAGGCAA AGCAUCACUA CAAUGACAAA UACGGGCGCA
3301 UUUGGACAAC AAUCAGGGGC AGUGUAUGUG GGGAACUACA GGGUGGUAAA
3351 UAGACAUCUA GCUACCAGUG CUGACUGGCA AAACUGUGUG UGGGAAAGUU
3401 ACAACAGAGA CCUCUUAGUG AGCACGACCA CAGCACAUGG AUGUGAUAUU
3451 AUAGCCAGAU GUCAGUGCAC AACGGGAGUG UACUUUUGUG CGUCCAAAAA
3501 CAAGCACUAC CCAAUUUCGU UUGAAGGACC AGGUCUAGUA GAGGUCCAAG
3551 AGAGUGAAUA CUACCCCAGG AGAUACCAAU CCCAUGUGCU UUUAGCAGCU
3601 GGAUUUUCCG AACCAGGUGA CUGUGGCGGU AUCCUAAGGU GUGAGCAUGG
3651 UGUCAUUGGC AUUGUGACCA UGGGGGGUGA AGGCGUGGUC GGCUUUGCAG
3701 ACAUCCGUGA UCUCCUGUGG CUGGAAGAUG AUGCAAUGGA ACAGGGAGUG
3751 AAGGACUAUG UGGAACAGCU UGGAAAUGCA UUCGGCUCCG GCUUUACUAA
3801 CCAAAUAUGU GAGCAAGUCA ACCUCCUGAA AGAAUCACUA GUGGGUCAAG
3851 ACUCCAUCUU AGAGAAAUCU CUAAAAGCCU UAGUUAAGAU AAUAUCAGCC
3901 UUAGUAAUUG UGGUGAGGAA CCACGAUGAC CUGAUCACUG UGACUGCCAC
3951 ACUAGCCCUU AUCGGUUGUA CCUCGUCCCC GUGGCGGUGG CUCAAACAGA
4001 AGGUGUCACA AUAUUACGGA AUCCCUAUGG CUGAACGCCA AAACAAUAGC
4051 UGGCUUAAGA AAUUUACUGA AAUGACAAAU GCUUGCAAGG GUAUGGAAUG
4101 GAUAGCUGUC AAAAUUCAGA AAUUCAUUGA AUGGCUCAAA GUAAAAAUUU
4151 UGCCAGAGGU CAGAGAAAAA CACGAGUUCC UGAACAGACU UAAACAACUC
4201 CCCUUAUUAG AAAGUCAGAU CGCCACAAUC GAGCAGAGCG CGCCAUCCCA
4251 AAGUGACCAG GAACAAUUAU UUUCCAAUGU CCAAUACUUU GCCCACUAUU
4301 GCAGAAAGUA CGCUCCCCUC UACGCAGCUG AAGCAAAGAG GGUGUUCUCC
4351 CUUGAGAAGA AGAUGAGCAA UUACAUACAG UUCAAGUCCA AAUGCCGUAU

| | | | | | |
|---|---|---|---|---|---|
| 4401 | UGAACCUGUA | UGUUUGCUCC | UGCACGGGAG | CCCUGGUGCC | GGCAAGUCGG |
| 4451 | UGGCAACAAA | CUUAAUUGGA | AGGUCGCUUG | CUGAGAAACU | CAACAGCUCA |
| 4501 | GUGUACUCAC | UACCGCCAGA | CCCAGAUCAC | UUCGACGGAU | ACAAACAGCA |
| 4551 | GGCCGUGGUG | AUUAUGGACG | AUCUAUGCCA | GAAUCCUGAU | GGGAAAGACG |
| 4601 | UCUCCUUGUU | CUGCCAAAUG | GUUUCCAGUG | UAGAUUUUGU | ACCACCCAUG |
| 4651 | GCUGCCCUAG | AAGAGAAAGG | CAUUCUGUUC | ACCUCACCGU | UUGUCUUGGC |
| 4701 | AUCGACCAAU | GCAGGAUCUA | UUAAUGCUCC | AACCGUGUCA | GAUAGCAGAG |
| 4751 | CCUUGGCAAG | GAGAUUUCAC | UUUGACAUGA | ACAUCGAGGU | UAUUUCCAUG |
| 4801 | UACAGUCAGA | AUGGCAAGAU | AAACAUGCCC | AUGUCAGUCA | AGACUUGUGA |
| 4851 | CGAUGAGUGU | UGCCCGGUCA | AUUUUAAAAA | GUGCUGCCCU | CUUGUGUGUG |
| 4901 | GGAAGGCUAU | ACAAUUCAUU | GAUAGAAGAA | CACAGGUCAG | AUACUCUCUA |
| 4951 | GACAUGCUAG | UCACCGAGAU | GUUUAGGGAG | UACAAUCAUA | GACAUAGCGU |
| 5001 | GGGGACCACG | CUUGAGGCAC | UGUUCCAGGG | ACCACCAGUA | UACAGAGAGA |
| 5051 | UCAAAAUUAG | CGUUGCACCA | GAGACACCAC | CACCGCCCGC | CAUUGCGGAC |
| 5101 | CUGCUCAAAU | CGGUAGACAG | UGAGGCUGUG | AGGGAGUACU | GCAAAGAAAA |
| 5151 | AGGAUGGUUG | GUUCCUGAGA | UCAACUCCAC | CCUCCAAAUU | GAGAAACAUG |
| 5201 | UCAGUCGGGC | UUUCAUUUGC | UUACAGGCAU | UGACCACAUU | UGUGUCAGUG |
| 5251 | GCUGGAAUCA | UAUAUAUAAU | AUAUAAGCUC | UUUGCGGGUU | UUCAAGGUGC |
| 5301 | UUAUACAGGA | GUGCCCAACC | AGAAGCCCAG | AGUGCCUACC | CUGAGGCAAG |
| 5351 | CAAAAGUGCA | AGGCCCUGCC | UUUGAGUUCG | CCGUCGCAAU | GAUGAAAAGG |
| 5401 | AACUCAAGCA | CGGUGAAAAC | UGAAUAUGGC | GAGUUUACCA | UGCUGGGCAU |
| 5451 | CUAUGACAGG | UGGGCCGUUU | UGCCACGCCA | CGCCAAACCU | GGGCCAACCA |
| 5501 | UCUUGAUGAA | UGAUCAAGAG | GUUGGUGUGC | UAGAUGCCAA | GGAGCUAGUA |
| 5551 | GACAAGGACG | GCACCAACUU | AGAACUGACA | CUACUCAAAU | UGAACCGGAA |
| 5601 | UGAGAAGUUC | AGAGACAUCA | GAGGCUUCUU | AGCCAAGGAG | GAAGUGGAGG |
| 5651 | UUAAUGAGGC | AGUGCUAGCA | AUUAACACCA | GCAAGUUUCC | CAACAUGUAC |
| 5701 | AUUCCAGUAG | GACAGGUCAC | AGAAUACGGC | UUCCUAAACC | UAGGUGGCAC |
| 5751 | ACCCACCAAG | AGAAUGCUUA | UGUACAACUU | CCCCACAAGA | GCAGGCCAGU |
| 5801 | GUGGUGGAGU | GCUCAUGUCC | ACCGGCAAGG | UACUGGGUAU | CCAUGUUGGU |
| 5851 | GGAAAUGGCC | AUCAGGGCUU | CUCAGCAGCA | CUCCUCAAAC | ACUACUUCAA |
| 5901 | UGAUGAGCAA | GGUGAAAUAG | AAUUUAUUGA | GAGCUCAAAG | GACGCCGGGU |
| 5951 | UUCCAGUCAU | CAACACACCA | AGUAAAACAA | AGUUGGAGCC | UAGUGUUUUC |
| 6001 | CACCAGGUCU | UUGAGGGGAA | CAAAGAACCA | GCAGUACUCA | GGAGUGGGGA |
| 6051 | UCCACGUCUC | AAGGCCAAUU | UUGAAGAGGC | UAUAUUUUCC | AAGUAUAUAG |
| 6101 | GAAAUGUCAA | CACACACGUG | GAUGAGUACA | UGCUGGAAGC | AGUGGACCAC |

6151 UACGCAGGCC AACUAGCCAC CCUAGAUAUC AGCACUGAAC CAAUGAAACU
6201 GGAGGACGCA GUGUACGGUA CCGAGGGUCU UGAGGCGCUU GAUCUAACAA
6251 CGAGUGCCGG UUACCCAUAU GUUGCACUGG GUAUCAAGAA GAGGGACAUC
6301 CUCUCUAAGA AGACUAAGGA CCUAACAAAG UUAAAGGAAU GUAUGGACAA
6351 GUAUGGCCUG AACCUACCAA UGGUGACUUA UGUAAAAGAU GAGCUCAGGU
6401 CCAUAGAGAA GGUAGCGAAA GGAAAGUCUA GGCUGAUUGA GGCGUCCAGU
6451 UUGAAUGAUU CAGUGGCGAU GAGACAGACA UUUGGUAAUC UGUACAAAAC
6501 UUUCCACCUA AACCCAGGGG UUGUGACUGG UAGUGCUGUU GGGUGUGACC
6551 CAGACCUCUU UUGGAGCAAG AUACCAGUGA UGUUAGAUGG ACAUCUCAUA
6601 GCAUUUGAUU ACUCUGGGUA CGAUGCUAGC UUAAGCCCUG UCUGGUUUGC
6651 UUGCCUAAAA AUGUUACUUG AGAAGCUUGG AUACACGCAC AAAGAGACAA
6701 ACUACAUUGA CUACUUGUGC AACUCCCAUC ACCUGUACAG GGAUAAACAU
6751 UACUUUGUGA GGGGUGGCAU GCCCUCGGGA UGUUCUGGUA CCAGUAUUUU
6801 CAACUCAAUG AUUAACAAUA UCAUAAUUAG GACACUAAUG CUAAAAGUGU
6851 ACAAAGGGAU UGACUUGGAC CAAUUCAGGA UGAUCGCAUA UGGUGAUGAU
6901 GUGAUCGCAU CGUACCCAUG GCCUAUAGAU GCAUCUUUAC UCGCUGAAGC
6951 UGGUAAGGGU UACGGGCUGA UCAUGACACC AGCAGAUAAG GGAGAGUGCU
7001 UUAACGAAGU UACCUGGACC AACGCCACUU UCCUAAAGAG GUAUUUUAGA
7051 GCAGAUGAAC AGUACCCCUU CCUGGUGCAU CCUGUUAUGC CCAUGAAAGA
7101 CAUACACGAA UCAAUUAGAU GGACCAAGGA UCCAAAGAAC ACCCAAGAUC
7151 ACGUGCGCUC ACUGUGUCUA UUAGCUUGGC AUAACGGGGA GCACGAAUAU
7201 GAGGAGUUCA UCCGUAAAAU UAGAAGCGUC CCAGUCGGAC GUUGUUUGAC
7251 CCUCCCCGCG UUUUCAACUC UACGCAGGAA GUGGUUGGAC UCCUUUUAGA
7301 UUAGAGACAA UUUGAAAUAA UUUAGAUUGG CUUAACCCUA CUGUGCUAAC
7351 CGAACCAGAU AACGGUACAG UAGGGGUAAA UUCUCCGCAU UCGGUGCGG

**8.** Rekombinante DNA nach Anspruch 6, **dadurch gekennzeichnet,** daß sie die Nukleotide 532 bis 2041 der Sequenz von Anspruch 7 enthält.

**532** **CAGCGGAAC CGACUACUUU**
**551 GGGUGUCCGU GUUUCAUUUU AUUCCUAUAC UGGCUGCUUA UGGUGACAAU**
**601 UGAGAGAUCG UUACCAUAUA GCUAUUGGAU UGGCCAUCCG GUGACUAAUA**

651 GAGCUAUUAU AUAUCCCUUU GUUGGGUUUA UACCACUUAG CUUGAAAGAG
701 GUUAAAACAU UACAAUUCAU UGUUAAGUUG AAUACAGCAA AAUGGGAGCU
751 CAAGUAUCAA CGCAAAAGAC UGGGGCACAU GAGACCAGGC UGAAUGCUAG
801 CGGCAAUUCC AUCAUUCACU ACACAAAUAU UAAUUAUUAC AAGGAUGCCG
851 CAUCCAACUC AGCCAAUCGG CAGGAUUUCA CUCAAGACCC GGGCAAGUUC
901 ACAGAACCAG UGAAAGAUAU CAUGAUUAAA UCACUACCAG CUCUCAACUC
951 CCCCACAGUA GAGGAGUGCG GAUACAGUGA CAGGGCGAGA UCAAUCACAU
1001 UAGGUAACUC CACCAUAACG ACUCAGGAAU GCGCCAACGU GGUGGUGGGC
1051 UAUGGAGUAU GGCCAGAUUA UCUAAAGGAU AGUGAGGCAA CAGCAGAGGA
1101 CCAACCGACC CAACCAGACG UUGCCACAUG UAGGUUCUAU ACCCUUGACU
1151 CUGUGCAAUG GCAGAAAACC UCACCAGGAU GGUGGUGGAA GCUGCCCGAU
1201 GCUUUGUCGA ACUUAGGACU GUUUGGGCAG AACAUGCAGU ACCACUACUU
1251 AGGCCGAACU GGGUAUACCG UACAUGUGCA GUGCAAUGCA UCUAAGUUCC
1301 ACCAAGGAUG CUUGCUAGUA GUGUGUGUAC CGGAAGCUGA GAUGGGUUGC
1351 GCAACGCUAG ACAACACCCC AUCCAGUGCA GAAUUGCUGG GGGGCGAUAC
1401 GGCAAAGGAG UUUGCGGACA AACCGGUCGC AUCCGGGUCC AACAAGUUGG
1451 UACAGAGGGU GGUGUAUAAU GCAGGCAUGG GGGUGGGUGU UGGAAACCUC
1501 ACCAUUUUCC CCCACCAAUG GAUCAACCUA CGCACCAAUA AUAGUGCUAC
1551 AAUUGUGAUG CCAUACACCA ACAGUGUACC UAUGGAUAAC AUGUUUAGGC
1601 AUAACAACGU CACCCUAAUG GUUAUCCCAU UUGUACCGCU AGAUUACUGC
1651 CCUGGGUCCA CCACGUACGU CCCAAUUACG GUCACGAUAG CCCCAAUGUG
1701 UGCCGAGUAC AAUGGGUUAC GUUUAGCAGG GCACCAGGGC UUACCAACCA
1751 UGAAUACUCC GGGGAGCUGU CAAUUUCUGA CAUCAGACGA CUUCCAAUCA
1801 CCAUCCGCCA UGCCGCAAUA UGACGUCACA CCAGAGAUGA GGAUACCUGG
1851 UGAGGUGAAA AACUUGAUGG AAAUAGCUGA GGUUGACUCA GUUGUCCCAG
1901 UCCAAAAUGU UGGAGAGAAG GUCAACUCUA UGGAAGCAUA CCAGAUACCU
1951 GUGAGAUCCA ACGAAGGAUC UGGAACGCAA GUAUUCGGCU UUCCACUGCA
2001 ACCAGGGUAC UCGAGUGUUU UUAGUCGGAC GCUCCUAGGA G

9. Rekombinante DNA nach Anspruch 6, **dadurch gekennzeichnet,** daß sie die Nukleotide 2289 bis 3600 der Sequenz von Anspruch 7 enthält.

2289 UA CCGCAGGGGG
2301 UUUUAUUACG UGCUGGUAUC AAACAAACAU AGUGGUCCCA GCGGAUGCCC
2351 AAAGCUCCUG UUACAUCAUG UGUUUCGUGU CAGCAUGCAA UGACUUCUCU
2401 GUCAGGCUAU UGAAGGACAC UCCUUUCAUU UCGCAGCAAA ACUUUUUCCA
2451 GGGCCCAGUG GAAGACGCGA UAACAGCCGC UAUAGGGAGA GUUGCGGAUA
2501 CCGUGGGUAC AGGGCCAACC AACUCAGAAG CUAUACCAGC ACUCACUGCU
2551 GCUGAGACGG GUCACACGUC ACAAGUAGUG CCGGGUGACA CUAUGCAGAC
2601 ACGCCACGUU AAGAACUACC AUUCAAGGUC CGAGUCAACC AUAGAGAACU
2651 UCCUAUGUAG GUCAGCAUGC GUGUACUUUA CGGAGUAUAA AAACUCAGGU
2701 GCCAAGCGGU AUGCUGAAUG GGUAUUAACA CCACGACAAG CAGCACAACU
2751 UAGGAGAAAG CUAGAAUUCU UUACCUACGU CCGGUUCGAC CUGGAGCUGA
2801 CGUUUGUCAU AACAAGUACU CAACAGCCCU CAACCACACA GAACCAAGAU
2851 GCACAGAUCC UAACACACCA AAUUAUGUAU GUACCACCAG GUGGACCUGU
2901 ACCAGAUAAA GUUGAUUCAU ACGUGUGGCA AACAUCUACG AAUCCCAGUG
2951 UGUUUUGGAC CGAGGGAAAC GCCCCGCCGC GCAUGUCCAU ACCGUUUUUG
3001 AGCAUUGGCA ACGCCUAUUC AAAUUUCUAU GACGGAUGGU CUGAAUUUUC
3051 CAGGAACGGA GUUUACGGCA UCAACACGCU AAACAACAUG GGCACGCUAU
3101 AUGCAAGACA UGUCAACGCU GGAAGCACGG GUCCAAUAAA AAGCACCAUU
3151 AGAAUCUACU UCAAACCGAA GCAUGUCAAA GCGUGGAUAC CUAGACCACC
3201 UAGACUCUGC CAAUACGAGA AGGCAAAGAA CGUGAACUUC CAACCCAGCG
3251 GAGUUACCAC UACUAGGCAA AGCAUCACUA CAAUGACAAA UACGGGCGCA
3301 UUUGGACAAC AAUCAGGGGC AGUGUAUGUG GGGAACUACA GGGUGGUAAA
3351 UAGACAUCUA GCUACCAGUG CUGACUGGCA AAACUGUGUG UGGGAAAGUU
3401 ACAACAGAGA CCUCUUAGUG AGCACGACCA CAGCACAUGG AUGUGAUAUU
3451 AUAGCCAGAU GUCAGUGCAC AACGGGAGUG UACUUUUGUG CGUCCAAAAA
3501 CAAGCACUAC CCAAUUUCGU UUGAAGGACC AGGUCUAGUA GAGGUCCAAG
3551 AGAGUGAAUA CUACCCCAGG AGAUACCAAU CCCAUGUGCU UUUAGCAGCU

10. Rekombinante DNA nach Anspruch 6, **dadurch gekennzeichnet,** daß sie die Nukleotide 6059 bis 7130 der Sequenz von Anspruch 7 enthält.

6059 UC AAGGCCAAUU UUGAAGAGGC UAUAUUUUCC AAGUAUAUAG
6101 GAAAUGUCAA CACACACGUG GAUGAGUACA UGCUGGAAGC AGUGGACCAC
6151 UACGCAGGCC AACUAGCCAC CCUAGAUAUC AGCACUGAAC CAAUGAAACU

6201 GGAGGACGCA GUGUACGGUA CCGAGGGUCU UGAGGCGCUU GAUCUAACAA
6251 CGAGUGCCGG UUACCCAUAU GUUGCACUGG GUAUCAAGAA GAGGGACAUC
6301 CUCUCUAAGA AGACUAAGGA CCUAACAAAG UUAAAGGAAU GUAUGGACAA
6351 GUAUGGCCUG AACCUACCAA UGGUGACUUA UGUAAAAGAU GAGCUCAGGU
6401 CCAUAGAGAA GGUAGCGAAA GGAAAGUCUA GGCUGAUUGA GGCGUCCAGU
6451 UUGAAUGAUU CAGUGGCGAU GAGACAGACA UUUGGUAAUC UGUACAAAAC
6501 UUUCCACCUA AACCCAGGGG UUGUGACUGG UAGUGCUGUU GGGUGUGACC
6551 CAGACCUCUU UUGGAGCAAG AUACCAGUGA UGUUAGAUGG ACAUCUCAUA
6601 GCAUUUGAUU ACUCUGGGUA CGAUGCUAGC UUAAGCCCUG UCUGGUUUGC
6651 UUGCCUAAAA AUGUUACUUG AGAAGCUUGG AUACACGCAC AAAGAGACAA
6701 ACUACAUUGA CUACUUGUGC AACUCCCAUC ACCUGUACAG GGAUAAACAU
6751 UACUUUGUGA GGGGUGGCAU GCCCUCGGGA UGUUCUGGUA CCAGUAUUUU
6801 CAACUCAAUG AUUAACAAUA UCAUAAUUAG GACACUAAUG CUAAAAGUGU
6851 ACAAAGGGAU UGACUUGGAC CAAUUCAGGA UGAUCGCAUA UGGUGAUGAU
6901 GUGAUCGCAU CGUACCCAUG GCCUAUAGAU GCAUCUUUAC UCGCUGAAGC
6951 UGGUAAGGGU UACGGGCUGA UCAUGACACC AGCAGAUAAG GGAGAGUGCU
7001 UUAACGAAGU UACCUGGACC AACGCCACUU UCCUAAAGAG GUAUUUUAGA
7051 GCAGAUGAAC AGUACCCCUU CCUGGUGCAU CCUGUUAUGC CCAUGAAAGA
7101 CAUACACGAA UCAAUUAGAU GGACCAAGGA

**11.** Mikroorganismus, **dadurch gekennzeichnet,** daß er eine rekombinante DNA nach einem der Ansprüche 6 bis 10 enthält.

**12.** E. coli VP4/2/3, DSM 5558.

**13.** E. coli, VP1.

**14.** E. coli $3D^{Pol}$.

**15.** Verfahren zur Herstellung eines Polypeptids nach einem der Ansprüche 1 bis 5, **dadurch gekennzeichnet,** daß man es aus ein Mikroorganismus nach einem der Ansprüche 11 bis 14 nah Aufschluß der Zellen gewinnt oder eine rekombinante DNA nach einem der Ansprüche 6 bis 10 in geeignete Wirtszellen einbringt und das Polypeptid aus dem Kulturmedium, gegebenenfalls nach Aufschluß der Zellen, gewinnt.

**16.** Verwendung eines Polypeptids nach einem der Ansprüche 1 bis 5, vorzugsweise einem der Ansprüche 2 bis 4 zur Erzeugung von gruppenspezifischen polyklonalen oder monoklonalen Antikörpern gegen Enteroviren.

**17.** Gruppenspezifischer immunologischer Nachweis von Enterovirus-Infektionen durch Untersuchung von Blut oder Serum von Patienten, **dadurch gekennzeichnet,** daß man in einem an sich bekannten immunologischen Test mindestens eines der Polypeptide nach einem der Ansprüche 1 bis 5 als Antigen zum Nachweis von Antikörpern gegen Enteroviren oder nach Anspruch 16 erzeugte Antikörper zum Nachweis von Virus-spezifischen Antigenen einsetzt.

# Fig.1

Aufbau des direkten ELISA für den Enterovirus-spezifischen Antigennachweis bzw. für den Antikörpernachweis

| Antigen - Nachweis | Antikörper - Nachweis | |
|---|---|---|
| Enzymkonjugat zum Nachweis von humanem-Immunglobulin | Enzymkonjugat zum Nachweis von humanem-Immunglobulin | 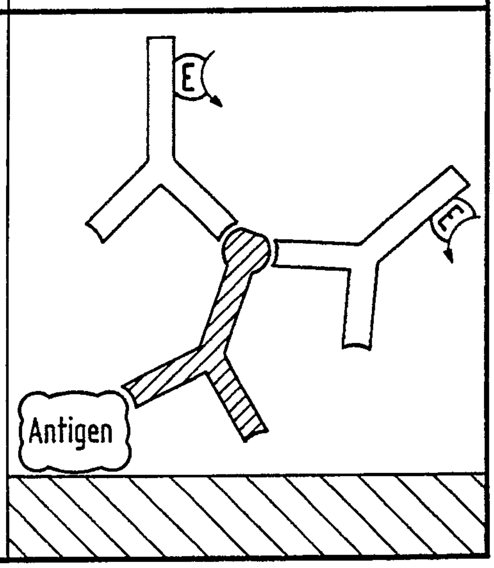  |
| polyklonales Antiserum (aVP4/2/3, aVP1 oder a3D$^{P01}$) | polyklonales Antiserum (aVP4/2/3, aVP1 oder a3D$^{P01}$) | |
| gebundenes Antigen aus Patientenserum | gebundenes Antigen aus Patientenserum | |
| Festphase | Festphase | |

# Fig. 2

Aufbau eines "Sandwich"-ELISA für den Enterovirus-spezifischen Antikörpernachweis

| Antikörper - Nachweis | |
|---|---|
| Enzymkonjugat zum Nachweis von humanem-Immunglobulin<br><br>Antikörper aus Patientenserum<br><br>aufgereinigtes Antigen aus infizierten Verozellen<br><br>gebundenes, polyklonales Antiserum (aVP4/2/3 oder aVP1 oder a3D$^{Pol}$)<br><br>Festphase | 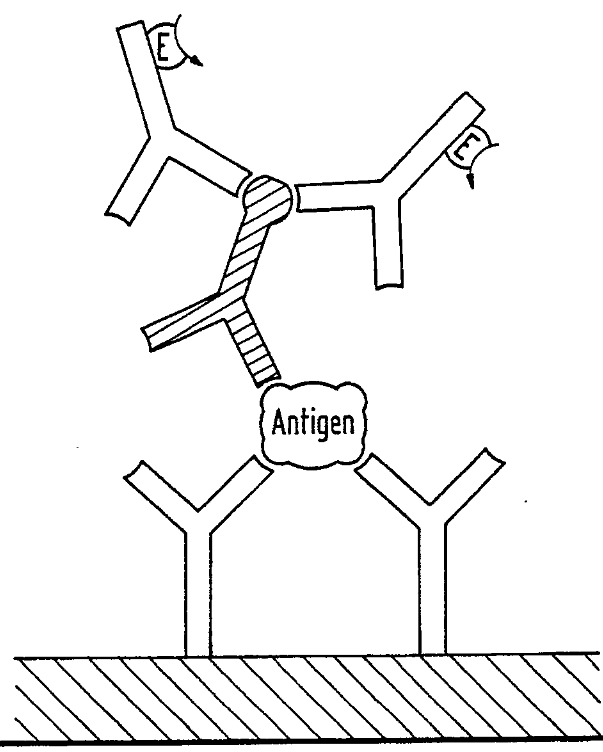 |

Europäisches Patentamt

# EUROPÄISCHER RECHERCHENBERICHT

Nummer der Anmeldung

**EP 90 12 2552**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DNA, Band 7, Nr. 5, 1988, Seiten 307-316, Mary Ann Liebert, Inc., Publishers; S. WERNER et al.: "Expression of coxsackievirus B3 capsid proteins in Escherichia coli and generation of virus-specific antisera" * Das ganze Dokument * | 1-17 | C 07 K 13/00<br>C 12 N 15/41<br>C 12 P 21/00<br>G 01 N 33/569 |
| X | VIROLOGY, Band 129, 1983, Seiten 431-442, Academic Press, Inc.; Th. MERTENS et al.: "Cross antigenicity among enteroviruses as revealed by immunoblot technique" * Das ganze Dokument * | 16,17 | |
| A | VIROLOGY, Band 156, 1987, Seiten 50-63, Academic Press, Inc.; A.M. LINDBERG et al.: "Genome of coxsackievirus B3" * Figur 2 * | 1-10 | |
| A | VIRUS RESEARCH, Band 3, 1985, Seiten 263-270, Elsevier Science Publishers B.V. (Biomedical Division); S. TRACY et al.: "Coxsackievirus B3: Primary structure of the 5' non-coding and capsid protein-coding regions of the genome" * Figur 1 * | 1-10 | |
| P,X | WO-A-8 909 790 (IMPERIAL COLLEGE) * Ansprüche * | 16,17 | |

RECHERCHIERTE SACHGEBIETE (Int. Cl.5)

C 12 N
A 61 K
C 07 K
G 01 N

Der vorliegende Recherchenbericht wurde für alle Patentansprüche erstellt

| Recherchenort | Abschlußdatum der Recherche | Prüfer |
|---|---|---|
| Den Haag | 28 Februar 91 | SKELLY J.M. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument